# FASCICULE DE BREVET EUROPEEN

(11) **EP 0 961 601 B1**
(45) Date de publication et mention de la délivrance du brevet: **16.07.2003**
(21) Numéro de dépôt: 98905408.5
(22) Date de dépôt: 06.02.1998
(51) Int. Cl.: A61F 6/06, A61F 6/08

(54) **PRESERVATIF FEMININ**
VERHÜTUNGSMITTEL FÜR FRAUEN
FEMALE CONDOM

(30) Priorité: 19.02.1997 LU 90030
(43) Date de publication de la demande: 08.12.1999
(73) Titulaire: Mediteam SPRL, 1040 Bruxelles (BE)
(72) Inventeur: COUVREUR, Chantal, B-1950 Kraainem (BE)
(74) Mandataire: Kihn, Pierre Emile Joseph
(86) Numéro de dépôt international: EP9800642
(87) Numéro de publication internationale: WO98036716

(56) Documents cités:
- WO-A-88/06432
- FR-A- 2 730 920
- US-A- 4 840 624
- US-A- 5 325 871

## Description

La présente invention concerne des préservatifs féminins.

Bien que les campagnes de prévention du SIDA recommandent l'usage du préservatif, une bonne partie des populations les plus exposées ne l'utilise pas. Il s'agit notamment :
- des jeunes,
- des couples dont l'homme ne sait pas aussi bien jouir avec un préservatif,
- des couples dont le conjoint infidèle ne peut pas demander subitement l'usage du préservatif sans provoquer de soupçons,
- des prostituées dont certains clients refusent l'usage pour des motifs divers.

Alors qu'on constate une stabilisation de l'incidence du SIDA chez les homosexuels ou les hommes bissexuels, le pourcentage de contamination dans la population hétérosexuelle est encore en augmentation - la majorité de ces cas sont dus à la pratique de relations sexuelles sans moyens de précaution.

Au cours de la XI ^{e} Conférence Internationale sur le SIDA (Vancouver 1996), une représentante brésilienne a montré que l'usage du préservatif traditionnel masculin, même lorsque la femme le désirait, était souvent rendu impossible par le refus du conjoint (LOUREIRO R., Condom use among young women of the urban poor population, who attend a public hospital of Porto Alegre, Brazil, XI International Conference on Aids, Vancouver July 7-12 1996, Th. C 4483 pp 329-330) et un groupe de chercheurs de la Harvard Médical School et de l'Organisation Mondiale de la Santé a estimé que l'usage d'un préservatif féminin donnait une plus grande liberté aux femmes pour négocier des relations sexuelles sans risques (PUROHIT A., TAMASHIRO H., STEINBERG J., KIESSLING A., CHAKRABORTY J., Prevention issues of maie and female condoms, XI International Conference on Aids, Vanouver July 7-12 1996, Th. C. 4464 p. 327).

D'autre part, on sait que les femmes sont plus vulnérables au SIDA, ainsi qu'aux autres maladies sexuellement transmissibles, que les hommes.

C'est pourquoi le rapport de la Commission Européenne sur l'état de la santé des femmes dans l'Union Européenne (1996) estime que, comme les femmes sont plus vulnérables que les hommes pour contracter cette maladie, "c'est sur elles que devraient être axés les efforts pour prévenir la propagation de cette maladie mortelle" (Report on the state of women's health in the European Community, Commission of the European Communities 1996)

En outre, on sait que les femmes sont habituellement de meilleurs agents de prévention.

Il existe actuellement sur le marché un certain nombre de préservatifs féminins. Le brevet EP 0 280 943 décrit un préservatif féminin constitué d'un dispositif tubulaire pour la protection contre le transfert de maladies sexuellement transmissibles, pouvant être inséré dans le vagin d'une utilisatrice, le préservatif féminin comprenant un tube à paroi mince et flexible ayant une extrémité fermée et une extrémité ouverte, ladite extrémité ouverte ayant un moyen pour l'étirer radialement qui empêche ledit tube de s'affaisser et qui maintient ladite ouverture ouverte.

Un des désavantages d'un tel préservatif féminin est que le risque d'une pénétration en dehors du préservatif est relativement élevé surtout en cas de contacts répétés.

Le brevet FR,A,2 730 920 présente un dispositif prophylactique pré-porté auto-introductif à la pénétration. Le dispositif est constitué d'un étui de rétention de forme générale tronconique, lequel est fermé à sa petite extrémité par une calotte sphérique, l'autre extrémité étant ouverte. L'extrémité ouverte est solidaire d'une plaque de maintien comportant une ouverture dont le périmètre est égal à celui de l'extrémité ouverte. La plaque est résistante pour pouvoir maintenir l'ouverture dudit étui sensiblement circulaire tout en restant flexible. L'ensemble est destiné à être assemblé par l'intermédiaire de la plaque de maintien à un support textile, tel un sous-vêtement.

Le but de la présente invention est de proposer un préservatif féminin diminuant le risque d'une pénétration en dehors du préservatif féminin lors du coït.

A cet effet, l'invention propose un préservatif féminin comprenant un tube à paroi mince et flexible ayant une extrémité fermée et une extrémité ouverte, ladite extrémité ouverte ayant un moyen pour étirer radialement ladite extrémité ouverte, ledit moyen pour étirer radialement ladite extrémité ouverte comprenant une collerette en matériau plus dur au toucher que le tube mince et flexible s'étendant perpendiculairement audit tube de façon à recouvrir la vulve pendant le coït caractérisé en ce que ladite collerette comprend une excroissance allongée en direction de l'anus. Elle recouvre donc la zone comprise entre la vulve et l'anus d'une utilisatrice pendant le coït.

Pour qu'un préservatif féminin puisse efficacement empêcher la transmission du virus du SIDA ou d'autres virus et germes, il est fondamental que, même lors de contacts répétés, le pénis ne puisse pas pénétrer le vagin en dehors du préservatif. C'est pourquoi, il est avantageux de prévoir un préservatif féminin dont la collerette comprend une excroissance ou languette allongée vers l'anus, qui se place parallèlement à la vulve et perpendiculairement à la partie tubulaire du préservatif.

La collerette complétée de la languette recouvrant la vulve de la femme maintient l'extrémité ouverte du préservatif féminin en place et empêche efficacement la transmission de maladies car le contact entre les fluides corporels de l'homme et de la femme est empêché. La collerette munie de la languette, en matériau plus dur au toucher, permet à l'homme de déterminer la zone d'entrée du préservatif féminin et diminue de cette façon le risque d'une pénétration en dehors du préservatif.

En cas de pénétration en-dehors. du préservatif féminin, le fait que la collerette est en un matériau plus dur au toucher permettra aux partenaires de se rendre compte rapidement du problème. Ceci constitue un avantage considérable par rapport aux préservatifs féminins connus n'ayant pas cette collerette plus dure au toucher et pour lesquels le pénis doit être guidé dans le tube et avec lesquels les partenaires ne se rendent compte de rien en cas de pénétration en-dehors du préservatif féminin.

Selon un premier mode de réalisation avantageux, le moyen pour étirer radialement ladite extrémité ouverte comprend un anneau qui a sensiblement le même diamètre que ledit tube et qui délimite la collerette par rapport au tube. L'extrémité ouverte du tube est maintenue ouverte par exemple à l'aide d'un anneau en matière plastique qui comprend sur son pourtour extérieur une collerette en matériau plus dur au toucher que le tube mince et flexible.

De préférence, l'anneau a un diamètre identique à celui d'un vagin. Cet anneau peut, selon un autre mode de réalisation, coulisser sur ledit tube.

Avantageusement, la collerette est fabriquée en un matériau plus rigide et/ou en un matériau plus épais que le tube mince et flexible.

Dans une réalisation préférée de l'invention, la longueur du tube à paroi mince et flexible est choisie de sorte que la collerette soit maintenue en contact avec la vulve. En vue d'augmenter le confort du préservatif féminin, il est préférable que la collerette soit maintenue en permanence appliquée contre les lèvres du vagin. Comme le vagin peut avoir des tailles différentes, il est préférable de mettre à la disposition des préservatifs féminins de différentes longueurs. Éventuellement, on pourrait aussi prévoir différentes largeurs de la collerette.

Dans une autre exécution avantageuse, ladite extrémité fermée comprend un moyen de retenue permettant de maintenir le préservatif féminin à l'intérieur du vagin pendant le rapport sexuel.

De préférence, ledit moyen de retenue est un anneau en un matériau semi-rigide ou en mousse. Ledit moyen de retenue peut être imprégné d'une matière spermicide et/ou antiseptique.

Pour les personnes particulièrement exposées au virus (partenaire séropositif), un diaphragme disposé à l'intérieur de la partie tubulaire ou un disque de fine mousse pourrait éviter le risque de perforation du préservatif.

Pour les personnes moins exposées à ce risque, un anneau ou un disque évidé en mousse ou un anneau semi-rigide pourrait suffire à maintenir le préservatif à l'intérieur du vagin pendant le rapport sexuel.

Selon un autre mode de réalisation avantageux, ledit moyen de retenue est insérable de manière amovible dans ladite extrémité fermée.

Le préservatif féminin peut également comprendre un moyen pour fixer ledit moyen de retenue au tube à paroi mince.

Avantageusement, le tube à paroi mince et flexible est fabriqué en un matériau polymère tel que le latex de caoutchouc naturel (NRL), le polyéthylène, le polyuréthane et les dérivés et mélanges de ceux-ci. Le préservatif féminin peut être lubrifié à l'intérieur et/ou à l'extérieur par un lubrifiant adéquat pour augmenter le confort.

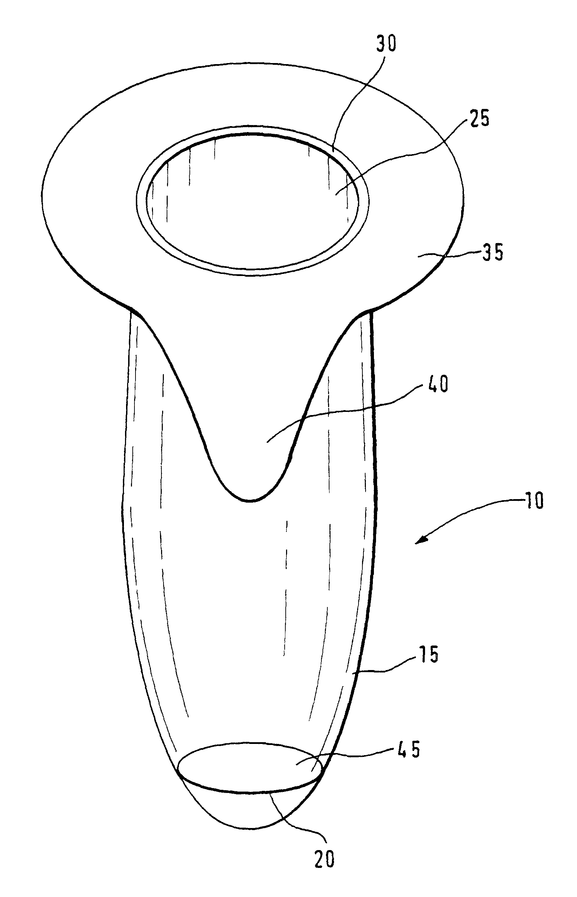

D'autres caractéristiques de l'invention sont décrites, à titre non limitatif, dans les exemples et en relation avec la figure qui montre un préservatif féminin.

Le préservatif féminin 10 pouvant être inséré dans le vagin d'une utilisatrice comprend un tube 15 à paroi mince et flexible ayant une extrémité fermée 20 et une extrémité ouverte 25. Celle-ci 25 éventuellement comprend un anneau 30 qui a sensiblement le même diamètre que ledit tube 15 et qui délimite la collerette 35 par rapport au tube 15. L'extrémité ouverte 25 du tube 15 est éventuellement maintenue ouverte à l'aide de cet anneau 30 en matière plastique qui comprend sur son pourtour extérieur la collerette 35 en matériau plus dur au toucher que le tube mince et flexible. La collerette 35 s'étend perpendiculairement audit tube 15 de façon à recouvrir la vulve pendant le coït. Le préservatif féminin 15 offre en outre une bonne protection contre la transmission de maladies sexuellement transmissibles ainsi qu'une protection efficace contre des grosseses involontaires. La collerette recouvrant la vulve et diminuant le risque d'une pénétration en-dehors du préservatif rend le préservatif féminin plus sur qu'un préservatif classique pour hommes. Contrairement aux préservatifs féminins connus, le présent préservatif féminin peut comprenre un anneau entre la collerette et le tube. Cet anneau diminue encore le risque d'une pénétration involontaire en-dehors du préservatif. Le préservatif féminin est maintenu immobile à l'intérieur du vagin pendant le coït, de même la collerette ne bouge en principe pas.

L'épaisseur du tube et de la collerette peuvent varier étant entendu que des épaisseurs de paroi et de collerette plus importantes diminuent la sensibilité lors du coït. Des épaisseurs typiques pour le tube se situent entre 20 et 100 microns tandis que la collerette peut être plus épaisse.

Bien entendu, l'épaisseur de la paroi doit être telle que la résistance aux contraintes mécaniques du préservatif féminin imposée par les normes en vigueur dans les différents pays soit assurée. Les dimensions du préservatif peuvent être telles qu'elles permettent le mouvement d'un pénis par rapport au préservatif féminin pendant l'acte sexuel.

La collerette 35 comprend une excroissance ou languette 40 allongée vers l'anus, qui se place parallèlement à la vulve et perpendiculairement à la partie tubulaire du préservatif. La languette 40 couvre la zone comprise entre la vulve et l'anus de l'utilisatrice pendant le coït.

Le préservatif féminin 10 peut être fabriqué en différentes longueurs c.-à-d. que la longueur du tube 15 peut être différente en fonction de la taille du vagin de l'utilisatrice, de sorte que pour chaque utilisatrice, la collerette 35 soit maintenue en contact avec la vulve. En général, la largeur de la collerette 35 se situe entre 1 et 4 cm et de préférence entre 1 et 2 cm. Bien entendu, la largeur de la collerette 35 ne doit pas nécessairement être uniforme sur son pourtour.

L'extrémité fermée 20 peut comprendre un moyen de retenue 45 permettant de maintenir le préservatif féminin 10 à l'intérieur du vagin pendant le rapport sexuel.

Le moyen de retenue 45 peut être un diaphragme, un disque de fine mousse ou un anneau en un matériau semi-rigide ou en mousse. Il peut être imprégné d'une matière spermicide et/ou antiseptique.

Le préservatif féminin 15 est fabriqué en un matériau polymère tel que le latex de caoutchouc naturel (NRL), le polyéthylène, le polyuréthane et les dérivés et mélanges de ceux-ci. Le préservatif féminin 15 peut être lubrifié à l'intérieur et/ou à l'extérieur par un lubrifiant adéquat pour augmenter le confort.

Le préservatif féminin peut être mis en place soit juste avant le coït soit déjà bien avant. Contrairement au préservatif masculin classique, il n'est pas nécessaire d'enlever le préservatif féminin directement après l'éjaculation.

## Revendications

1. Préservatif féminin (10) pour la protection contre le transfert de maladies sexuellement transmissibles pouvant être inséré dans le vagin d'une utilisatrice, le préservatif féminin comprenant un tube (15) à paroi mince et flexible ayant une extrémité fermée (20) et une extrémité ouverte (25), ladite extrémité ouverte (25) ayant un moyen pour *étirer radialement* ladite extrémité ouverte (25), ledit moyen pour étirer radialement ladite extrémité ouverte (25) comprenant une collerette (35) en matériau plus dur au toucher que le tube (15) mince et flexible s'étendant perpendiculairement audit tube (15) de façon à recouvrir la vulve pendant le coït **caractérisé en ce que** ladite collerette (35) comprend une excroissance (40) allongée en direction de l'anus.

2. Préservatif féminin selon la revendication 1, **caractérisé en ce que** le moyen pour étirer radialement ladite extrémité ouverte (25) comprend un anneau (30) qui a sensiblement le même diamètre que ledit tube (15) qui délimite la collerette (35) par rapport au tube (15).

3. Préservatif féminin selon l'une quelconque des revendications précédentes, **caractérisé en ce que** la collerette (35) est fabriquée en un matériau plus rigide que le tube (15) mince et flexible.

4. Préservatif féminin selon l'une quelconque des revendications précédentes, **caractérisé en ce que** la collerette (35) est en matériau plus épais que le tube (15) mince et flexible.

5. Préservatif féminin selon l'une quelconque des revendications précédentes, **caractérisé en ce que** la longueur du tube (15) à paroi mince et flexible est choisie de sorte que la collerette (35) soit maintenue en contact avec la vulve.

6. Préservatif féminin selon l'une quelconque des revendications précédentes, **caractérisé en ce que** ladite extrémité fermée (20) comprend un moyen de retenue (45) permettant de maintenir le préservatif féminin (10) à l'intérieur du vagin pendant le rapport sexuel.

7. Préservatif féminin selon la revendication 6, **caractérisé en ce que** ledit moyen de retenue (45) est un anneau (30) en un matériau semi-rigide ou en mousse.

8. Préservatif féminin selon l'une quelconque des revendications 7 ou 8, **caractérisé en ce que** ledit moyen de retenue (45) est imprégné d'une matière spermicide et/ou antiseptique.

9. Préservatif féminin selon l'une quelconque des revendications 6 à 8, **caractérisé en ce que** ledit moyen de retenue (45) est insérable de manière amovible dans ladite extrémité fermée (20).

10. Préservatif féminin selon la revendication 6, **caractérisé en ce qu'**il (10) comprend en outre un moyen pour fixer ledit moyen de retenue (45) au tube (15) à paroi mince.

11. Préservatif féminin selon l'une quelconque des revendications précédentes, **caractérisé en ce que** le tube (15) à paroi mince et flexible est fabriqué en un matériau polymère tel que le latex de caoutchouc naturel (NRL), le polyéthylène, le polyuréthane et les dérivés et mélanges de ceux-ci.

## Patentansprüche

1. Frauenkondom (10) zum Schutz vor der Übertragung sexuell übertragbarer Krankheiten, das in die Vagina einer Benutzerin eingeführt werden kann, wobei das Frauenkondom ein dünnwandiges, flexibles Rohr (15) mit einem geschlossenen Ende (20) und einem offenen Ende (25) umfasst, wobei das offene Ende (25) ein Mittel aufweist, um das offene Ende (25) radial zu strecken, wobei das Mittel zum radialen Strecken des offenen Endes (25) einen Kragen (35) aus einem sich härter anfühlenden Material als das dünne, flexible Rohr (15) aufweist, der sich quer zu diesem Rohr (15) erstreckt, um die Vulva während des Coitus zu bedecken, **dadurch gekennzeichnet, dass** der Kragen (35) einen zum After verlängerten Fortsatz (40) aufweist.

2. Frauenkondom nach Anspruch 1, **dadurch gekennzeichnet, dass** das Mittel zum radialen Strecken des offenen Endes (25) einen Ring (30) aufweisen kann, der weitgehend den gleichen Durchmesser wie das Rohr (15) hat und den Kragen (35) in Bezug auf das Rohr (15) abgrenzt.

3. Frauenkondom nach einem der vorhergehenden Ansprüche, **dadurch gekennzeichnet, dass** der Kragen (35) aus einem steiferen Material als das dünne, flexible Rohr (15) gefertigt ist.

4. Frauenkondom nach einem der vorhergehenden Ansprüche, **dadurch gekennzeichnet, dass** der Kragen (35) aus dickerem Material als das dünne, flexible Rohr (15) ist.

5. Frauenkondom nach einem der vorhergehenden Ansprüche, **dadurch gekennzeichnet, dass** die Länge des dünnwandigen, flexiblen Rohrs (15) so gewählt ist, dass der Kragen (35) mit der Vulva in Berührung bleibt.

6. Frauenkondom nach einem der vorhergehenden Ansprüche, **dadurch gekennzeichnet, dass** das geschlossene Ende (20) ein Haltemittel (45) aufweist, mit dem das Frauenkondom (10) während des sexuellen Kontaktes im Innern der Vagina gehalten wird.

7. Frauenkondom nach Anspruch 6, **dadurch gekennzeichnet, dass** das Haltemittel (45) ein Ring (30) aus einem halbsteifen Material oder aus Schaumstoff ist.

8. Frauenkondom nach einem der Ansprüche 7 oder 8, **dadurch gekennzeichnet, dass** das Haltemittel (45) mit einer spermiziden und/oder antiseptischen Substanz imprägniert ist.

9. Frauenkondom nach einem der Ansprüche 6 bis 8, **dadurch gekennzeichnet, dass** das Haltemittel (45) herausnehmbar in das geschlossene Ende (20) eingesetzt werden kann.

10. Frauenkondom nach Anspruch 6, **dadurch gekennzeichnet, dass** es (10) zusätzlich ein Mittel zum Befestigen des Haltemittels (45) am dünnwandigen Rohr (15) aufweist.

11. Frauenkondom nach einem der vorhergehenden Ansprüche, **dadurch gekennzeichnet, dass** das dünnwandige, flexible Rohr (15) aus einem Polymermaterial wie Naturgummilatex (NLR), Polyethylen, Polyurethan und deren Derivaten und Gemischen gefertigt ist.

## Claims

1. Female condom (10) for protection against the transfer of sexually transmitted diseases capable of being inserted in the vagina of a user, the female condom comprising a flexible thin-walled tube (15) having a closed end (20) and an open end (25), the said open end (25) having means for stretching the said open end (25) radially, the said means for stretching the said open end (25) radially comprising a collarette (35), made of a material harder to the touch than the flexible thin tube (15), extending in a direction perpendicular to the said tube (15) in such a way as to cover the vulva during coitus **characterised in that** the said collarette (35) has a protuberance (40) elongated in the direction of the anus.

2. Female condom according to Claim 1, **characterised in that** the means for stretching the said open end (25) radially may comprise a ring (30) with roughly the same diameter as the said tube (15) and delimiting the collarette (35) with respect to the tube (15).

3. Female condom according to any one of the preceding claims, **characterised in that** the collarette (35) is made from a more rigid material than the thin flexible tube (15).

4. Female condom according to any one of the preceding claims, **characterised in that** the collarette (35) is made from a thicker material than the thin flexible tube (15).

5. Female condom according to any one of the preceding claims, **characterised in that** the length of the flexible thin-walled tube (15) is chosen so that the collarette (35) is maintained in contact with the vulva.

6. Female condom according to any one of the preceding claims, **characterised in that** the said closed end (20) incorporates a means for retention (45) enabling the female condom (10) to be held inside the vagina during sexual intercourse.

7. Female condom according to Claim 6, **characterised in that** the said means for retention (45) is a ring (30) made of a semi-rigid material or of foam rubber.

8. Female condom according to Claims 7 or 8, **characterised in that** the said means for retention (45) may be impregnated with a spermicidal and/or antiseptic substance.

9. Female condom according to any one of Claims 6 to 8, **characterised in that** the said means for retention (45) is capable of being inserted in the said closed end (20) in such a way that it can be removed.

10. Female condom according to Claim 6 9, **characterised in that** it (10) also incorporates a means for fixing the said means for retention (45) to the thin-walled tube (15).

11. Female condom according to any one of the preceding claims, **characterised in that** the flexible thin-walled tube (15) is made from a polymeric material such as natural rubber latex (NRL), polyethylene, polyurethane and derivatives and mixtures of these materials.
